# EUROPEAN PATENT SPECIFICATION

(11) **EP 4 324 456 B1**
(45) Date of publication and mention of the grant of the patent: **21.10.2026**
(21) Application number: 22787615.8
(22) Date of filing: 15.04.2022
(51) Int. Cl.: A61K 31/136, A61K 9/127, A61P 35/00, A61P 35/04, A61K 9/00, A61K 9/1271

(54) **MITOXANTRONE HYDROCHLORIDE LIPOSOME FOR USE IN THE TREATMENT OF UROTHELIAL CARCINOMA**
LIPOSOMALES MITOXANTRONHYDROCHLORID ZUR VERWENDUNG BEI DER BEHANDLUNG VON UROTHELKARZINOMEN
MITOXANTRONE CHLORHYDRATE LIPOSOMAL POUR UTILISATION DANS LE TRAITEMENT DU CARCINOME UROTHÉLIAL

(30) Priority: 16.04.2021 CN 202110410490
(43) Date of publication of application: 21.02.2024
(73) Proprietor: CSPC Zhongqi Pharmaceutical Technology (Shijiazhuang) Co., Ltd., Shijiazhuang, Hebei 050035 (CN)
(72) Inventor: LI, Chunlei, Shijiazhuang, Hebei 050035 (CN); LIU, Yanping, Shijiazhuang, Hebei 050035 (CN); LIANG, Min, Shijiazhuang, Hebei 050035 (CN); LI, Mengmeng, Shijiazhuang, Hebei 050035 (CN); LI, Tong, Shijiazhuang, Hebei 050035 (CN); YANG, Hua, Shijiazhuang, Hebei 050035 (CN); WANG, Shixia, Shijiazhuang, Hebei 050035 (CN); YANG, Sensen, Shijiazhuang, Hebei 050035 (CN)
(74) Representative: Gassner, Birgitta
(86) International application number: PCT/CN2022/086959
(87) International publication number: WO 2022/218393

(56) References cited:
- EP-A1- 4 098 251
- WO-A1-2018/048752
- WO-A1-2020/011189
- WO-A1-2021/160115
- CN-A- 103 622 909
- CN-A- 105 287 383
- CN-A- 109 937 046
- CN-A- 110 711 178
- US-A1- 2020 197 534
- MOMEKOVA D. ET AL: "Physicochemical and biopharmaceutical characterization of dipalmitoyl phosphatidylcholine liposomes sterically stabilized by copolymers bearing short blocks of lipid-mimetic units", vol. 6, no. 3, 21 December 2009 (2009-12-21), GB, pages 591 - 601, XP093190546, ISSN: 1744-683X, Retrieved from the Internet <URL:https://pubs.rsc.org/en/content/articlepdf/2010/sm/b917275k> DOI: 10.1039/B917275K
- ADIGBLI D K ET AL: "Photochemical internalisation of chemotherapy potentiates killing of multidrug-resistant breast and bladder cancer cells", vol. 97, no. 4, 31 July 2007 (2007-07-31), London, pages 502 - 512, XP093191212, ISSN: 0007-0920, Retrieved from the Internet <URL:http://www.nature.com/articles/6603895> DOI: 10.1038/sj.bjc.6603895
- MOUSSA MOHAMMAD JAD ET AL: "Revisiting Treatment of Metastatic Urothelial Cancer: Where Do Cisplatin and Platinum Ineligibility Criteria Stand?", BIOMEDICINES, vol. 12, no. 3, 26 February 2024 (2024-02-26), Basel, pages 519, XP093191152, ISSN: 2227-9059, DOI: 10.3390/biomedicines12030519
- SHI YONGPING,SU ZHIGUI,XIAO YANYU,SUN MINJIE,PING QINENG,ZONG LI: "Preparation and characterization of dual-targeting nanostructure lipid carriers loading mitoxantrone hydrochloride for bone tumor", JOURNAL OF CHINA PHARMACEUTICAL UNIVERSITY, vol. 44, no. 4, 15 August 2013 (2013-08-15), pages 321 - 327, XP055976721, ISSN: 1000-5048, DOI: 10.11665/j.issn.1000-5048.20130407
- POUJOL, SYLVAIN; TILLEUL, PATRICK; ASTRE, CECILE; MARTEL, PASCAL; FABBRO, MICHEL; PINGUET, FREDERIC: "Effent of Mitoxantrone Liposomes on Multidrug-Resistant Breast Cancer Cells", ANTICANCER RESEARCH, vol. 19, no. 4B, 31 December 1999 (1999-12-31), GR , pages 3327 - 3331, XP009529784, ISSN: 0250-7005
- ANONYMOUS: "CSPC's mitoxantrone hydrochloride liposome injection passed the registration site inspection and workshop GMP compliance inspection", 29 December 2020 (2020-12-29), pages 1 - 4, XP055976743, Retrieved from the Internet <URL:http://finance.china.com.cn/industry/medicine/20201229/5464701.shtml> [retrieved on 20221101]

## Description

The present invention claims to enjoy the priority of a prior application with the patent application number of 202110410490.2 and the title of "use of mitoxantrone hydrochloride liposome", which was submitted to the China National Intellectual Property Administration on April 16, 2021.

The present patent application cites PCT application of WO2008/080367A1 filed on December 29, 2007.

### Technical field

The invention belongs to the field of anti-tumor, and specifically relates to the use of mitoxantrone hydrochloride liposome, such as the use in preparation of a drug for treating urothelial carcinoma.

### Background art

Urothelial carcinoma is a multi-origin malignant tumor originating from the urothelium, including renal pelvis cancer, ureteral cancer, bladder cancer and urethral cancer, etc., which is the most common urinary system tumor. Globally, the incidence of bladder-derived tumors ranked 12^{th} among all solid tumors in 2018, accounting for 2.1% of total tumor deaths (Freddie, Bray, Jacques, Ferlay, Isabelle, & Soerjomataram, et al. (2018). Global cancer statistics 2018: globocan estimates of incidence and mortality worldwide for 36 cancers in 185 countries. CA: a cancer journal for clinicians.). In China, bladder-derived tumors ranked out of 10th in all tumors in 2015; ranked 7^{th} in male, 6.2/100,000 people; ranked out of 10^{th} in female; the standardized mortality rate ranked 14^{th} among all tumors, 1.96/100,000 people (Zheng Rongshou, Sun Kexin, Zhang Siwei, et al. Analysis of the prevalence of malignant tumors in China in 2015. Chinese Journal of Oncology, January 2019, Volume 41, Issue 1). The ratio of male to female incidence in bladder cancer is approximately 3:1 (Freddie, Bray, Jacques, Ferlay, Isabelle, & Soerjomataram, et al. (2018). Global Cancer statistics 2018: globocan estimates of incidence and mortality worldwide for 36 cancers in 185 countries. CA: a cancer journal for clinicians.).

For the first-line treatment of unresectable locally advanced or metastatic urothelial carcinoma, for the patients who are tolerant to cisplatin, cisplatin-containing chemotherapy regimens, such as GC (gemcitabine + cisplatin), MVAC (methotrexate + vinblastine + adriamycin + cisplatin) is the primely recommended program. Patients who cannot tolerate cisplatin can consider carboplatin-containing regimens, such as gemcitabine + carboplatin regimen. For other patients, PD-1 inhibitors such as pembrolizumab or atezolizumab, and chemotherapy drugs such as gemcitabine + paclitaxel or gemcitabine alone, can be used. PD-1 inhibitors are primely recommended in patients with PD-L1 expression. For the second-line treatment of patients, the current treatment methods include immunotherapy, targeted therapy, ADC drug therapy and chemotherapy. There is currently no standard treatment for the third-line and above, and corresponding chemotherapy, targeted therapy, and biological therapy should be selected according to the use of front-line drugs.

In conclusion, patients who have failed in treatment with platinum-containing chemotherapy regimens and PD-1 inhibitors lack effective follow-up treatments, and new drugs are urgently needed to be explored.

Mitoxantrone hydrochloride is an anthraquinone chemotherapy drug. The FDA-approved indications for mitoxantrone hydrochloride ordinary injection are multiple sclerosis, prostate cancer and acute myeloid leukemia; it has been approved in China for patients suffering from lymphoma, leukemia and breast cancer with a recommended dose of 12-14 mg/m² for adults as a single drug, once every 3-4 weeks; or 4-8 mg/m², once a day for 3-5 days, with an interval of 2-3 weeks. The combined dose is 5-10 mg/m² once. Mitoxantrone is an anthracycline drug, and the main adverse reactions are mainly cardiotoxicity, myelosuppression and gastrointestinal reactions etc. Among them, the myelosuppression and gastrointestinal reactions can be solved by giving appropriate drugs, but cardiotoxicity often brings serious consequences and is the most serious adverse reaction of anthracyclines. Clinical studies and practical observations have shown that the cardiotoxicity resulting from the anthracyclines is mostly progressive and irreversible, especially the first use of anthracyclines can easily cause cardiac damage. Chronic Dose Accumulation Limiting Toxicity - cardiotoxicity is a common concern for clinicians. Therefore, although the indication for breast cancer is approved, mitoxantrone is not recommended as a treatment for breast cancer by authoritative clinical medication guidelines. For urothelial carcinoma, bone and soft tissue sarcoma, mitoxantrone has never been approved for these two indications.

Different tumors have different drug sensitivities. Existing studies have shown that when the same drug is used to treat different indications, its dosage regimen may be different. For example, Doxil (doxorubicin hydrochloride liposome) has been approved by the FDA for three indications, namely: (1) ovarian cancer, the recommended dose is 50 mg/m², administered intravenously once every 4 weeks; (2) Kaposi's sarcoma, the recommended dose is 20 mg/m², administered intravenously once every 3 weeks; (3) Multiple myeloma, the recommended dose is 30 mg/m², intravenously administered on the fourth day after bortezomib administration. Another example is Abraxane (paclitaxel for injection [albumin-bound]) which is also approved by the FDA for three indications, (1) metastatic breast cancer: the recommended dose is 260 mg/m², intravenous infusion for 30 minutes, once every 3 weeks; (2)Non-small cell lung cancer: the recommended dose is 100 mg/m², intravenous infusion for 30 minutes, a course of treatment every 21 days, administered on the 1^{st} day, the 8^{th} day and the 15^{th} day respectively; for the injection on the 1^{st} day Carboplatin is administered immediately after administration of paclitaxel (albumin-bound), once every 21 days; (3) Pancreatic cancer: the recommended dose is 125 mg/m², intravenous infusion over 30-40 minutes, with a cycle of 28 days , once administered on the 1^{st}, 8^{th} and 15^{th} day respectively, and gemcitabine was administered immediately after each administration of paclitaxel for injection (albumin-bound). For another example, the starting dose of AmBisome (Amphotericin B liposome for injection) for the treatment of the following indications is: (1) empirical treatment: the recommended dose is 3 mg/kg/day; (2) systemic fungal infections (Aspergillus, Candida , Cryptococcus): the recommended dose is 3-5 mg/kg/day; (3) Cryptococcal meningitis in HIV-infected patients: the recommended dose is 6 mg/kg/day (day 1 - day 5), 3 mg/kg/day (day 4, day 21); (4) Immunocompromised patients with visceral leishmaniasis: 4 mg/kg/day (day 1 - day 5), 4 mg/kg/day (day 10, day 17, day 24, day 31, day 38); dosage and administration data are customized according to the actual situation of the patient to achieve maximum efficacy and minimum toxicity or adverse reactions. It can be seen that there are differences in the safe and effective doses of the same drug for different indications. Dosage and administration data should be customized according to the specific disease and the actual situation of the patient, so as to achieve the maximum efficacy and minimum toxicity or adverse reactions, and achieve the effect of safe and effective treatment of the disease. For the special dosage form of mitoxantrone liposome, which is different from ordinary injections, its absorption, distribution and metabolism after entering the body are very complicated. For the treatment of different indications, especially in the treatment of different tumors, it is difficult to simply deduce from one indication to another.

Currently, mitoxandrone hydrochloride liposome injection has been used in first-line studies in the field of hematologic malignancies. Related clinical studies for leukemia, multiple myeloma, and diffuse large B-cell lymphoma are also underway. In the field of solid tumors, studies have been conducted on breast cancer, small cell lung cancer, liver cancer, reproductive system tumors, head an neck tumors, urothelial carcinoma, sarcoma, gastric cancer, colorectal cancer, and lung cancer (China.com.cn. "CSPC's Mitoxantrone Hydrochloride Liposome Injection Passed the Registration Site Inspection and Workshop GMP Compliance Inspection." December 29, 2020. http://finance.china.com.cn/industry/medicine/20201229/5464701.shtml.)

Therefore, it is necessary to systematically study whether mitoxantrone liposome is suitable for the treatment of urothelial carcinoma and to clarify its safety and effective dose, so as to provide reference for clinical treatment.

### Summary of the invention

The invention is set out in the appended set of claims.

The invention relates to mitoxantrone hydrochloride liposome for use in a method for treating urothelial carcinoma, the method is to administer a therapeutically effective amount of mitoxantrone hydrochloride liposome to a patient suffering from urothelial carcinoma; wherein the urothelial cancer is locally advanced or metastatic urothelial carcinoma; and wherein the patient suffering therefrom is subjected to treatment-failure via platinum containing chemotherapy regimens and/or PD-1 inhibitors. The invention also relates to mitoxantrone hydrochloride liposome as the only active ingredient for use in a method for treating urothelial carcinoma, the method is to administer a therapeutically effective amount of mitoxantrone hydrochloride liposome to a patient suffering from urothelial carcinoma; wherein the urothelial cancer is locally advanced or metastatic urothelial carcinoma; and wherein the patient suffering therefrom is subjected to treatment-failure via platinum containing chemotherapy regimens and/or PD-1 inhibitors.

Preferably, mitoxantrone hydrochloride liposome is used as the only active ingredient for preparing a drug for treating urothelial carcinoma.

Preferably, the drug is in the form of injection, including liquid injection, powder for injection, tablet for injection, and the like.

Preferably, the drug is a liquid injection.

According to an embodiment of the present invention, when the drug is a liquid injection, based on mitoxantrone, the medicine contains mitoxantrone in amount of 0.5-5 mg/ml, preferably 1-2 mg/ml, more preferably 1 mg/ml.

The urothelial carcinoma is locally advanced or metastatic urothelial carcinoma wherein the patient suffering therefrom is subjected to treatment-failure via platinum-containing chemotherapy regimen and/or PD-1 inhibitor therapy, or locally advanced or metastatic urothelial carcinoma wherein the patient suffering therefrom is subjected to treatment-failure via platinum-containing chemotherapy and refuses PD-1 inhibitor therapy.

Preferably, based on mitoxantrone, the therapeutically effective amount means that the dose of mitoxantrone is 8-30 mg/m², more preferably 12-20 mg/m² or 16-30 mg/m². Specific examples are 12 mg/m², 14 mg/m², 16 mg/m², 18 mg/m², and 20 mg/m².

Preferably, the mode of administration of the present invention is intravenous administration. Preferably, the administration cycle is once per 3 weeks. Preferably, the treatment given to the patient is 6-8 cycles. Preferably, for each intravenous administration, the infusion administration time of the liposome pharmaceutical preparation is 30 min-120 min, preferably 60 min-120 min, further preferably 60±15 min.

In some embodiments, the liposome is in the form of injection, including liquid injection, powder for injection, tablet for injection, and the like. When the drug is a liquid injection, based on mitoxantrone, the liposome contains mitoxantrone in amount of 0.5-5 mg/ml, preferably 1-2 mg/ml, more preferably 1 mg/ml.

In some embodiments, the therapeutically effective amount of the liposome (based on mitoxantrone) is 8-30 mg/m², more preferably 12-20 mg/m² or 16-30 mg/m². Specific examples are 12 mg/m², 14 mg/m², 16 mg/m², 18 mg/m², and 20 mg/m².

In some embodiments, the liposome is administered intravenously. Preferably, the administration cycle is once per 3 weeks. Preferably, the treatment given to the patient is 6-8 cycles. Preferably, for each intravenous administration, the infusion administration time of the liposome is 30 min-120 min, preferably 60 min-120 min, further preferably 60±15 min.

In the context of the present invention, the doses are based on mitoxantrone unless otherwise specified.

In the context of the present invention, the mitoxantrone hydrochloride liposome can be prepared by using methods known in the art, and can be mitoxantrone hydrochloride liposome prepared by any method disclosed in the prior art, for example, the method disclosed in WO2008/080367A1.

In some embodiments, the drug or mitoxantrone hydrochloride liposome has one or more of the following properties:
(i) mitoxantrone hydrochloride liposome has a particle size of about 30-80 nm, such as about 35-75 nm, about 40-70 nm, about 40-60 nm, or about 60 nm;
(ii) mitoxantrone hydrochloride forms insoluble precipitate with multivalent counter ions (e.g. sulfate, citrate or phosphate) within the liposome;
(iii) the phospholipid bilayer in the mitoxantrone hydrochloride liposome contains a phospholipid with a phase transition temperature (Tm) higher than body temperature, such that the phase transition temperature of the liposome is higher than body temperature, for example, the phospholipid is selected from hydrogenated soy lecithin, phosphatidylcholine, hydrogenated ovolecithin, lecithin bis palmitate, lecithin bis stearate, or any combination thereof;
(iv) the phospholipid bilayer of mitoxantrone hydrochloride liposome contains hydrogenated soy lecithin, cholesterol, and distearoylphosphatidylethanolamine modified with polyethylene glycol 2000 (DSPE-PEG2000).
(v) the phospholipid bilayer in the mitoxantrone hydrochloride liposome contains hydrogenated soy lecithin, cholesterol, and distearoylphosphatidylethanolamine modified with polyethylene glycol 2000 in a mass ratio of about 3:1:1, mitoxantrone hydrochloride forms insoluble precipitate with multivalent acid ions in the liposome, and the particle size of the mitoxantrone hydrochloride liposome in the drug is about 60 nm.
(vi) the mitoxantrone hydrochloride liposome is the mitoxantrone hydrochloride liposome with the national medicine approval number H20220001.

In the context of the present invention, the mitoxantrone hydrochloride liposome has a particle size of about 30-80 nm and contains: 1) the active ingredient mitoxantrone, which can forms an insoluble precipitate with multivalent counter ions in the liposome, and 2) the phospholipid bilayer contains phospholipid with a phase transition temperature (Tm) higher than body temperature, so that the phase transition temperature of liposome is higher than body temperature. The phospholipid with a Tm higher than body temperature is phosphatidylcholine, hydrogenated soy lecithin, hydrogenated ovolecithin, lecithin bis palmitate, lecithin bis stearate, or any combination thereof, and the particle size is about 35-75 nm, preferably 40-70 nm, further preferably 40-60 nm, particularly preferably 60 nm. Preferably, the phospholipid bilayer contains hydrogenated soy lecithin, cholesterol and distearoylphosphatidylethanolamine modified with polyethylene glycol 2000 in a mass ratio of 3:1:1, the particle size is about 60 nm. The counter ion is the sulfate ion. Preferably, the phospholipid bilayer of the liposome contains hydrogenated soy lecithin, cholesterol and distearoylphosphatidylethanolamine modified with polyethylene glycol 2000 in a mass ratio of 3:1:1, and the particle size is about 40-60nm, the counter ion is sulfate ion. The weight ratio of HSPC : Chol : DSPE-PEG2000 : mitoxantrone in the liposome is 9.58:3.19:3.19:1.

In the context of the present invention, the mitoxantrone liposome is prepared as follows: HSPC (hydrogenated soy lecithin), Chol (cholesterol) and DSPE-PEG2000 (distearoylphosphatidylethanolamine modified with polyethylene glycol 2000) are weighed in a mass ratio of (3:1:1) and dissolved in 95% ethanol to obtain a clear solution. The ethanol solution of phospholipid is mixed with 300 mM ammonium sulfate solution, and hydrated by oscillating at 60-65 °C for 1 h to obtain heterogeneous multilamellar liposome. The particle size of the liposome is then reduced using a microfluidic device. The obtained sample is diluted 200 times with 0.9% NaCl solution and detected by NanoZS. The average particle size of the particles was about 60 nm, and the main peak was concentrated between 40 and 60 nm. The ammonium sulfate in the outer phase of the blank liposome is then removed by using an ultrafiltration device, and the outer phase is replaced with 290 mM sucrose and 10 mM glycine to form a transmembrane ammonium sulfate gradient. According to the ratio of lipid-drug ratio of 16:1, mitoxantrone hydrochloride solution (10 mg/mL) is added to blank liposome, and the drug is loaded at 60-65 °C. After incubation for about 1 h, the encapsulation efficiency is demonstrated to be about 100% by using gel exclusion chromatography. The weight ratio of HSPC : Chol : DSPE-PEG2000 : mitoxantrone is 9.58:3.19:3.19:1, and the osmotic pressure of the sucrose-glycine solution is close to the physiological value.

It should be understood that various technical details and parameters in the above-mentioned exemplary preparation methods can be adjusted and determined within a reasonable range by those skilled in the art. For example, the amino acid species that can substitute glycine in the outer phase to form the transmembrane ammonium sulfate gradient include, but are not limited to, histidine, asparagine, glutamic acid, leucine, proline, alanine. For another example, the mass ratio of HSPC, Chol and DSPE-PEG2000 can be adjusted appropriately. For another example, the lipid-drug ratio parameter for preparing the specific liposome pharmaceutical preparation, those skilled in the art can design, test and finally obtain a suitable lipid-drug ratio, so as to improve the drug load as much as possible and reduce the amount of drug leakage. For the mitoxantrone hydrochloride liposome formulation according to the application, a wide range of lipid-drug ratios can be used, for example, as low as 2:1 or as high as 30:1, 40:1 or 50:1, and more suitable ratio of lipid to drug may be about (15-20):1, such as about 15:1, 16:1, 17:1, 18:1, 19:1 or 20:1. Therefore, several advantageous properties of the liposomal formulations of mitoxantrone hydrochloride described above are more important, and the methods to achieve these properties are diverse.

The first-line, second-line or more than second-line drugs for treating urothelial carcinoma described in the present invention refer to drugs approved by drug administrations in China or abroad (such as the United States, the European Union, Japan, South Korea, etc.) for use in the first-line, second-line or more than second-line drugs for treating urothelial carcinoma including but not limited to: taxanes, PD-1 inhibitors, ifosfamide, etc. approved by FDA.

### Beneficial technical effects

The applicant of the present invention unexpectedly found that mitoxantrone hydrochloride liposome has better therapeutic effect on urothelial carcinoma and can be used for preparing related medicines.

### Specific embodiment

The technical solutions of the present invention will be described in further detail below with reference to specific embodiments. It should be understood that the following examples are only for illustrating and explaining the present invention, and should not be construed as limiting the protection scope of the present invention. All technologies implemented based on the above content of the present invention are covered within the intended protection scope of the present invention.

Unless otherwise stated, the starting materials and reagents used in the following examples are commercially available or can be prepared by known methods.

The following abbreviations are used in the present invention:
CR: Complete response, the specific definition is based on the "Response Evaluation Criteria in Solid Tumors (RECIST1.1)" commonly used in international research.
PR: Partial response, the specific definition is based on the "Response Evaluation Criteria in Solid Tumors (RECIST1.1)" commonly used in international research.
SD: stable disease, the specific definition is based on the "Response Evaluation Criteria in Solid Tumors (RECIST1.1)" commonly used in international research.
PD: progressive disease, the specific definition is based on the "Response Evaluation Criteria in Solid Tumors (RECIST 1.1)" commonly used in international research.$Objective response rate \left(ORR\right) = \left(CR+PR\right) / total number of evaluable cases * 100 % .$$Disease control rate \left(DCR\right) = \left(CR+PR+SD\right) / total number of evaluable cases * 100 % .$

In order to investigate the effect of mitoxantrone hydrochloride liposome in the treatment of urothelial carcinoma, breast cancer, bone and soft tissue sarcoma, the inventors carried out clinical research. Mitoxantrone hydrochloride liposome can effectively treat urothelial carcinoma, breast cancer, bone and soft tissue sarcoma. Compared with ordinary mitoxantrone hydrochloride injection, it has better treating effect and fewer adverse reactions. The mitoxantrone hydrochloride liposome injection used in the following examples was provided by CSPC Pharmaceutical Group Co. Ltd. Zhongnuo Pharmaceutical (Shijiazhuang) Co., Ltd. (national medicine approval number H20220001).

### Example 1 Phase II clinical trial for evaluating the efficacy and safety of mitoxantrone hydrochloride liposome injection in the treatment of unresectable locally advanced or metastatic urothelial carcinoma

This is an open, multicenter Phase II study in which subjects included will receive mitoxantrone hydrochloride liposome injection to evaluate the efficacy and safety of mitoxantrone hydrochloride liposome injection for unresectable locally advanced or metastatic urothelial carcinoma.

### I. Design of experiments

### 1. Test process

Forty subjects with unresectable locally advanced or metastatic urothelial carcinoma were planned to enroll. The study population was mainly subjects who were subjected to treatment-failure via platinum-containing chemotherapy regimen and PD-1 inhibitor therapy (including those who did not receive PD-1 inhibitor therapy); those who were intolerant to cisplatin and required systemic therapy. The study included a screening period, a treatment period, and a follow-up period. The screening period was 28 days, and qualified subjects were screened to enter the treatment period. During the treatment period, mitoxantrone hydrochloride liposome injection of 20 mg/m² monotherapy was given, every 3 weeks as a cycle (q3w), and was administered at the first day of each cycle for 6 cycles. The follow-up period was started after treatment. During the follow-up period, safety follow-up after the last administration (day 28 after the last administration) and survival follow-up (once every 6 weeks after the last administration) were conducted. Safety evaluation was carried out from the first dose to day 28 after the last administration, and safety evaluation was required before each cycle of administration to decide whether to administer the drug in the next cycle. Tumor evaluation was performed once every two cycles according to RECIST 1.1 criteria throughout the study period until disease progression, death or adopting new antitumor therapy.

### 2. Study Duration

The study included a screening period of 4 weeks (28 days), a treatment period of 6 cycles (18 weeks), a last administration safety follow-up visit, 4 weeks (28 days) after the end of dosing, followed by a survival follow-up visit once every 6 weeks. The duration for each patient was about 12 months.

40 subjects were planned to enroll in this study, and the whole cycle of this study was 18 to 24 months.

### II. Test population:

Subjects who met all the following inclusion criteria and did not have any one of the exclusion criteria were eligible for inclusion in this clinical study.

### (1) Selection criteria

1) The subjects voluntarily participate in the research and sign the informed consent;
2) Age ≥ 18 years old, male or female;
3) Histologically confirmed urothelial carcinoma;
4) Subjects who are subjected to treatment-failure via platinum-containing chemotherapy regimen and PD-1 inhibitor therapy (including those who did not receive PD-1 inhibitor therapy); those who are intolerant to cisplatin and require systemic therapy;
5) There is at least one evaluable lesion that meets the definition of RECIST 1.1 at baseline;
6) ECOG score of 0 to 1;
7) The laboratory test values of the subjects meet the following requirements:
   · Absolute neutrophil count (ANC) ≥ 1.5x10⁹/L (within 1 week before the laboratory inspection, without receiving G-CSF whitening treatment);
   · Hemoglobin (Hb) > 9.0 g/dL (within 1 week before laboratory inspection, without red blood cell transfusion);
   · Platelets ≥ 75x10⁹ /L (within 1 week before the laboratory inspection, without receiving platelet transfusion);
   · Creatinine ≤ 1.5x ULN;
   · Total bilirubin ≤ 1.5x ULN (for subjects with liver metastases, ≤3x ULN);
   · Alanine aminotransferase (AST) / aspartate aminotransferase (ALT) ≤3x ULN (for subjects with liver metastases, ≤5x ULN);
   · Coagulation function: prothrombin time (PT), international normalized ratio (INR) ≤ 1.5x ULN (for subjects who receive anticoagulant drugs such as warfarin, INR ≤ 3 is allowed);
8) The subjects and their partners take effective contraceptive measures during the trial period to 6 months after the end of the last medication (for example: combined hormones (including estrogen and progesterone) combined with ovulation suppression, progesterone contraception combined with ovulation inhibition, intrauterine device, intrauterine hormone release system, bilateral vas ligation, bilateral tubal ligation, avoiding sexual behavior, etc.); female subjects have negative urine or blood HCG (except for menopause and hysterectomy);

### (2) Exclusion criteria

1) Severe allergy to mitoxantrone or liposome drugs;
2) Subjects with brain or meningeal metastases;
3) Previous allogeneic organ transplantation or allogeneic bone marrow transplantation;
4) Survival period < 12 weeks;
5) Active hepatitis B (HbsAg or HBcAb positive and HBV DNA ≥ 2000 IU/mL), active hepatitis C (HCV antibody positive and HCV RNA higher than the lower limit of the detection value of the research center), HIV antibody positive patients;
6) Active bacterial infection or fungal infection or viral infection requiring intravenous infusion within 1 week before the first drug administration;
7) Those who have received any anti-tumor therapy within 4 weeks before the first administration (2 weeks before administration for traditional Chinese medicine or Chinese patent medicine);
8) Those who have received treatment with other clinical trial drugs within 4 weeks before the first administration;
9) Those who have undergone major surgery within 3 months before the first administration, or are planning to undergo major surgery during the study period;
10) There is previous anti-tumor treatment toxicity of > grade 1 (except for hair loss, pigmentation or other toxicity that is considered to have no safety risk to the subjects in the study);
11) Serious thrombosis or embolic events within the past 6 months, such as cerebrovascular accident (including transient ischemic attack), pulmonary embolism, etc.;
12) Other malignant active tumors within the past 3 years; except for locally curable cancers that have been cured, such as basal or squamous cell skin cancer or in situ prostate, cervical or breast cancer;
13) Abnormal cardiac function, including:
   · Long QTc syndrome or QTc interval >480 ms;
   · Complete left bundle branch block, degree II or III atrioventricular block (except for treatment by pacemaker implantation);
   · Severe, uncontrolled cardiac arrhythmias requiring medical treatment;
   · History of chronic congestive heart failure, NYHA ≥ grade 3;
   · Cardiac ejection fraction less than 50% within 6 months;
   · Valvular heart disease with CTCAE > grade 2;
   · Uncontrollable hypertension (defined as multiple measurements of systolic blood pressure >150 mmHg or diastolic blood pressure >100 mmHg under medication control);
   · History of myocardial infarction, unstable angina, severe pericardial disease, ECG evidence of acute ischemia or severe conduction system abnormalities within 6 months prior to screening.
14) Adriamycin or other anthracycline therapy has been received, and the cumulative dose of adriamycin exceeds 350mg/m² (anthracycline equivalent dose calculation: 1mg adriamycin = 2mg epirubicin = 2mg pirarubicin = 0.5 mg idarubicin = 0.45 mg mitoxantrone; excluding adriamycin liposome);
15) Pregnant or lactating women;
16) Suffering from any serious and/or uncontrollable disease, which, as determined by investigators, other diseases that may affect the patient's participation in this study (including but not limited to, uncontrolled diabetes, kidney disease requiring dialysis, severe liver disease, life-threatening autoimmune and bleeding disorders, substance abuse, neurological disorders, etc.);
17) Other conditions unsuitable to participate judged by investigators.

### (3) Criteria for withdrawal from treatment and withdrawal from research

The Subject must stop receiving the investigational drug treatment if any of the following conditions occurs during the study:
1) The subject has intolerable toxicity, and the investigator evaluates that the risks of continuing to receive the treatment outweigh the benefits;
2) Concomitant diseases that occur during treatment do not allow subsequent continued administration;
3) Disease progression assessed by imaging;
4) There is clinically assessed disease progression or major protocol deviation, or poor compliance of the subject, and it is judged by the investigator that the continued treatment with the treatment will not benefit;
5) The subject is pregnant;
6) Death;
7) Meet any one of the withdrawal criteria.

All subjects who withdraw from treatment are required to continue follow-up in accordance with the study plan, except for subjects who discontinue treatment due to death or who meet any one of the following criteria for withdrawal from the study.

Subjects have the right to withdraw from the study at any time for any reason. Subjects will be withdrawn from the study process if any one of the following occurs:
1) Lost to follow-up;
2) The subject or family members (if the subject himself cannot make a decision) request to withdraw from the trial;
3) Termination of the study;
4) Other.

### 3. Research results

For the subjects who were subjected to treatment-failure via platinum-containing chemotherapy regimen and PD-1 inhibitor therapy (including those who did not receive PD-1 inhibitor therapy), and subjects who were intolerant to cisplatin and had undergone systemic therapy, there were not subsequently relatively effective therapies, it was urgently needed to explore new drugs. The inventors of the present invention used mitoxantrone liposome. After the drug entered the human body through intravenous infusion, it had the effects of sustained release, targeting, detoxification, and synergism. Compared with ordinary mitoxantrone injection, liposome preparation could be used in a higher dose, which could not only improve the effectiveness, but also reduce the incidence of adverse reactions, and the ideal therapeutic effect could be obtained only with a single drug treatment.

The research of the present invention showed that in at least 4 subjects (2 subjects subjected to 2 cycles of treatment, 2 subjects subjected to 1 cycle of treatment), for SD there were 2 cases (1 subject subjected to 1 cycle of treatment, 1 subject subjected to 2 cycles of treatment), DCR was 50% (2/4). Mitoxantrone liposome had good efficacy in patients with urothelial carcinoma, especially those who were subjected to treatment-failure via platinum-containing chemotherapy regimen and PD-1 inhibitor therapy. The 2 subjects who were evaluated as SD continued the treatment and obtained better curative effect.

This regime would improve the treatment regime for urothelial carcinoma, lay the foundation for the combination of drugs to impact first-line and second-line treatment, change the traditional treatment mode of urothelial carcinoma, and look forward to the application of similar high-efficiency and low-toxic chemotherapy drugs in the field of advanced solid tumors.

### Example 2 (reference example)

### Phase II clinical trial of mitoxantrone hydrochloride liposome injection in the treatment of HER-2 negative advanced breast cancer

This is an open-label, multicenter Phase II study in which subjects included will receive mitoxantrone hydrochloride liposome injection to evaluate the efficacy and safety of mitoxantrone hydrochloride liposome injection for HER-2 negative advanced breast cancer.

### I. Design of experiments

### 1. Test process

This study was divided into screening period, treatment period, and follow-up period. The screening period was 28 days, and eligible patients were screened to enter the treatment period. During the treatment period, the subjects received mitoxantrone hydrochloride liposome injection in 20 mg/m², 3 weeks as a cycle, 6 cycles. After the completion of 6 cycles of treatment, the investigator and the sponsor would determine whether to continue administration according to the benefits and the risks of the patients. Follow-up included end-of-treatment visit (day 28 after last administration) and survival visit (once every 6 weeks after end-of-treatment visit), and all subjects were followed up by telephone or visit until death.

Tumor evaluation: According to RECIST 1.1 criteria, tumor evaluation was performed every 2 cycles during the treatment period and every 6 weeks during the follow-up period until disease progression or new antitumor therapy.

Safety evaluation: Vital signs, physical examination, body weight, ECOG performance score, 12-lead electrocardiogram, echocardiography, and laboratory tests were completed before each cycle of treatment and at the end of treatment visit. Routine blood tests were performed on D8 and D15 of each cycle.

### 2. Study Duration

The study included a screening period of 4 weeks (28 days), a treatment period of 6 cycles (18 weeks), an end-of-treatment visit (day 28 after the last administration), followed by a survival follow-up once every 6 weeks until the patient was lost to follow-up or died. The duration for each patient was about 12 months.

73 subjects were enrolled in this study, and the whole cycle of this study is about 36 months.

### II. Test population:

Subjects who met all the following inclusion criteria and did not have any one of the exclusion criteria were eligible for inclusion in this clinical study.

### (1) Selection criteria:

Subjects must meet all the following criteria:
1) Voluntarily participate in the study and sign the informed consent form;
2) Age 18 to 75 years old (including 18 years old and 75 years old);
3) HER-2 negative breast cancer confirmed by histopathology (including immunohistochemical HER-2 0 or 1+, immunohistochemical HER-2 2+, which needs to be confirmed negative by in situ hybridization);
4) Inoperable locally advanced or recurrent/metastatic breast cancer wherein the patient suffering therefrom is subjected to disease progression after first-line or above chemotherapy;
5) Hormone receptor-negative or hormone receptor-positive breast cancer that is not suitable for endocrine therapy or resistant to endocrine therapy;
6) The therapy including at least one anthracycline and one taxane was received in the past (subjects who did not use anthracycline due to high risk factors of cardiotoxicity in the past can be accepted), and up to four chemotherapy regimens are received;
7) At least one measurable lesion at baseline (RECIST 1.1 criteria);
8) ECOG score of 0 to 2;
9) Good organ function (there is no blood transfusion or growth factor supportive therapy within 2 weeks before the first application of the study drug), including:
   · Absolute neutrophil count (ANC) ≥ 1.5×10⁹ /L;
   · Hemoglobin (Hb) ≥ 90 g/L;
   · Platelets ≥ 90×10⁹ / L;
   · Creatinine ≤ 1.5 times the upper limit of normal value;
   · Total bilirubin ≤ 1.5 times the upper limit of normal value;
   · Alanine aminotransferase (AST) and aspartate aminotransferase (ALT) ≤ 3 times the upper limit of normal value (≤5 times the upper limit of normal value in patients with liver metastases);
10) The pregnancy test result is negative, and the subject of childbearing age promises to take effective contraceptive measures or abstinence from the start of the study to 6 months after the last administration of the study;
11) Have good compliance and be willing to cooperate with follow-up.

### (2) Exclusion criteria:

Subjects meeting either of the following criteria will be excluded from this trial:
1) Severe allergy to mitoxantrone or liposome;
2) Suffering from other malignant tumors in the past 3 years, excluding cervical carcinoma in situ, skin basal cell carcinoma or squamous carcinoma that has been radically cured;
3) Subjects with brain metastases or meningeal metastases;
4) Active hepatitis B (HbsAg positive and HBV DNA ≥ 2000 IU/mL), active hepatitis C (HCV antibody positive and HCV RNA higher than the lower limit of the detection value of the research center), HIV antibody positive subjects;
5) Expected survival time < 3 months;
6) The cumulative dose of anthracyclines received in the past is converted to doxorubicin > 350 mg/m² (anthracycline equivalent dose calculation: 1mg doxorubicin = 2 mg epirubicin = 2 mg pirarubicin = 2 mg daunorubicin = 0.5 mg idarubicin = 0.45 mg mitoxantrone, excluding adriamycin liposome);
7) The toxicity of previous anti-tumor therapy has not recovered to ≤ grade 1 (except for hair loss, pigmentation or other toxicity that is considered to have no safety risk to the subjects in the study);
8) Abnormal cardiac function includes:
   · Long QTc syndrome or QTc interval > 480ms;
   · Complete left bundle branch block, degree II/III atrioventricular block;
   · Severe, uncontrolled cardiac arrhythmias requiring medical treatment;
   · History of chronic congestive heart failure and NYHA ≥ grade 3;
   · Cardiac ejection fraction less than 50% within 6 months prior to screening;
   · Valvular heart disease with CTCAE ≥ grade 3;
   ·History of myocardial infarction, unstable angina, severe ventricular arrhythmia, severe pericardial disease, ECG evidence of acute ischemic or active conduction system abnormalities within 6 months prior to screening.
9) Uncontrollable hypertension (under drug control, multiple measurements of systolic blood pressure ≥ 160 mmHg or diastolic blood pressure ≥ 100 mmHg);
10) Active bacterial infection, fungal infection, or viral infection requiring intravenous infusion therapy within 1 week before the first administration;
11) Any anti-tumor therapy (including chemotherapy, radiotherapy, molecular targeted therapy, immunotherapy, endocrine therapy) is received within 4 weeks before the first administration, and received an immunomodulator as an adjuvant therapy for malignant tumor is received within 2 weeks before the first administration, any anti-tumor Chinese patent medicine is received within 2 weeks before the first administration (except Chinese patent medicines for strengthening the body and Chinese patent medicines for alleviating symptoms).
12) Those who have received other clinical research drugs within 4 weeks before the first administration;
13) Those who have undergone major surgery within 12 weeks before the first administration, or are planning to undergo major surgery during the study period;
14) Deep vein thrombosis or arterial embolism within the past 6 months, including but not limited to superior/inferior vena cava thrombosis, lower extremity deep vein thrombosis, and pulmonary embolism;
15) Lactating women;
16) Suffering from any serious and/or uncontrollable disease, which, as determined by investigators, other diseases that may affect the patient's participation in this study (including but not limited to uncontrolled diabetes, kidney disease requiring dialysis, severe liver disease, life-threatening autoimmune and bleeding disorders, substance abuse, neurological disorders, etc.);
17) Other conditions unsuitable to participate judged by investigators.

### (3) Criteria for withdrawal from treatment and withdrawal from research

The Subject must stop receiving the investigational drug treatment if any of the following conditions occurs during the study:
1) The subject has intolerable toxicity, and the investigator evaluates that the risks of continuing to receive the treatment outweigh the benefits;
2) Disease progression assessed by imaging;
3) There is clinically assessed disease progression or major protocol deviation, or poor compliance of the subject, and the investigator judges that it is not beneficial to continue receiving the treatment;
4) The subject is pregnant;
5) Death;
6) Meet any one of the withdrawal criteria.

All subjects who withdraw from treatment are required to continue follow-up in accordance with the study plan, except for those who discontinue treatment due to death or who meet any of the following criteria for withdrawal from the study.

Subjects have the right to withdraw from the study at any time for any reason. Subjects will be withdrawn from the study process if any of the following occurs:
1) Lost to follow-up;
2) Subjects withdraw their informed consent or subjects or their family members request to withdraw from the trial;
3) Termination of the study;
4) Other.

### 3. Research results

There was an urgent need to explore new treatments for advanced breast cancer wherein the subject suffering therefrom was subjected to treatment-failure or recurrence via anthracyclines and taxanes.

The study of the present invention showed that, among at least the 20 evaluable cases, for PR there were 6 cases (1 case subjected to 7 cycles of treatment, 3 cases subjected to 6 cycles of treatment, 1 case subjected to 5 cycles of treatment, 1 case subjected to 4 cycles of treatment), for SD there were 8 cases (2 cases subjected to 6 cycles of treatment, 2 cases subjected to 5 cycles of treatment, 1 case subjected to 4 cycles of treatment, 1 case subjected to 3 cycles of treatment, and 2 cases subjected to 2 cycles of treatment). ORR was 30% (6/20), DCR was 70% (14/20).

Mitoxantrone liposome had good curative effect on breast cancer patients, especially on patients with advanced breast cancer who had been subjected to treatment-failure or recurrence via anthracycline and taxane therapy in the past. The 14 subjects who were evaluated as PR and SD continued the treatment and obtained better curative effect.

The inventor's previous clinical research included mitoxantrone hydrochloride monotherapy in the treatment of recurrent/metastatic breast cancer. There were 30 cases in the experimental group and the control group respectively. The experimental group was given mitoxantrone hydrochloride liposome injection in 20 mg/m², every 4 weeks as a cycle. The control group was given mitoxantrone hydrochloride injection in 14 mg/m², every 4 weeks as a cycle. Results: In the experimental group, for PR there were 4 cases, for SD there were 11 cases, ORR was 13.3% (4/30), DCR was 50% (15/30); in the control group, for PR there were 2 cases, for SD there were 7 cases, ORR was 6.7% (2/30), DCR was 30% (9/30).

The inventors of the present invention used mitoxantrone liposomes. After the drug entered the human body through intravenous infusion, it had the effects of sustained release, targeting, detoxification and synergism. Not only is the dosage higher than that of ordinary injections, but it was also a single drug treatment. It could not only improve the effectiveness, but also reduce the incidence of adverse reactions.

### Example 3 (reference example)

### Phase Ib clinical trial of mitoxantrone hydrochloride liposome injection in the treatment of advanced bone and soft tissue sarcoma wherein the subject suffering therefrom has failed at least one prior line of therapy

This is an open-label, multi-center Phase Ib study in which subjects included will receive mitoxantrone hydrochloride liposome injection to evaluate the efficacy and safety of mitoxantrone hydrochloride liposome injection for metastatic or unresectable bone and soft tissue sarcoma wherein the subject suffering therefrom has failed at least one prior line of therapy.

### I. Design of experiments

### 1. Test process

The study included a screening period, a treatment period, and a follow-up period.

The screening period was 28 days, and eligible subjects were screened to enter the treatment period. During the treatment period, subjects received mitoxantrone hydrochloride liposome injection in 20 mg/m² on the first day, 3 weeks as a treatment cycle (q3w), in a total of 6 cycles. For subjects who had completed 6 cycles of treatment, according to the risks and benefits of the subjects, the investigator and the sponsor discussed to determine whether to continue the administration. During the follow-up period, safety follow-up after the last administration (day 28±7 after the last administration) and survival follow-up (once every 6 weeks after the last administration) were carried out.

Safety evaluation was carried out from the first administration to day 28 after the last administration. Safety evaluation was required before each cycle of administration to decide whether to administer in the next cycle. During the entire study period, tumor evaluation was performed according to the RESIST 1.1 criteria. The tumor evaluation was performed every two cycles during the treatment period, and the follow-up period was performed every 6 weeks until disease progression, death, or new antitumor therapy.

### 2. Study Duration

The study included a screening period of 4 weeks (28 days), a treatment period of 6 cycles (18 weeks), a safety follow-up visit after last administration, at 4 weeks (28 days) after the end of dosing, followed by a survival follow-up once every 6 weeks, the duration for each patient was about 12 months.

No less than 50 subjects were enrolled in this study, and the whole cycle of this study was 24 to 36 months.

### II. Test population:

Subjects who met all the following inclusion criteria and did not have any of the exclusion criteria were eligible for inclusion in this clinical study.

### (1) Selection criteria:

Subjects must meet all of the following criteria:
1) The subjects voluntarily participate in the research and sign the informed consent;
2) Age ≥ 18 years old, male or female;
3), Osteosarcoma or unspecified soft tissue sarcoma (excluded in the types of soft tissue sarcoma: highly chemosensitive sarcoma such as embryonal/acinar rhabdomyosarcoma, Ewing sarcoma, chemotherapy extremely insensitive sarcoma such as alveolar soft part sarcoma, extraosseous myxoid chondrosarcoma, sarcomas that require special treatment (such as gastrointestinal stromal tumor, invasive fibromatosis, etc.), confirmed by histopathology;
4) Metastatic or unresectable osteosarcoma or soft tissue sarcoma wherein the subject suffering therefrom has failed at least one prior line of therapy ;
5) There is at least one measurable lesion that meets the definition of RECIST 1.1 at baseline;
6) ECOG score of 0 to 1;
7) Laboratory inspection values must meet the following standards:
   · Absolute neutrophil count (ANC) ≥ 2.0x10⁹/L (within 1 week before the laboratory inspection, without receiving G-CSF whitening treatment);
   · Hemoglobin (Hb) ≥ 110g/L (without receiving red blood cell transfusion therapy within 1 week before the laboratory inspection);
   · Platelets ≥ 100x 10⁹/L (without receiving platelet transfusion therapy within 1 week before the laboratory inspection);
   · Creatinine ≤ 1.5x ULN;
   · Total bilirubin ≤ 1.5x upper limit of normal (ULN) (subjects with liver metastases, ≤ 3.0x ULN);
   · Alanine aminotransferase (AST)/aspartate aminotransferase (ALT) ≤ 3.0x ULN (subjects with liver metastases, ≤ 5.0x ULN);
   · Coagulation function: prothrombin time (PT), international normalized ratio (INR) ≤ 1.5x ULN (for subjects who receive anticoagulant drugs such as warfarin, INR ≤ 3 is allowed)
8) Female subjects with negative HCG of urine or blood (except menopause and hysterectomy);
9) Subjects and their partners agree to take effective contraceptive measures during the trial and within 6 months after the end of the last medication (for example: combined hormones (including estrogen and progesterone) combined with ovulation suppression, progesterone contraception combined with ovulation suppression, intrauterine device, intrauterine hormone releasing system, bilateral vas ligation, bilateral tubal ligation, vasectomy, avoiding sexual behavior, etc.);

### (2) Exclusion criteria:

Subjects meeting either of the following criteria will be excluded from this trial:
1) Severe allergy to mitoxantrone or liposome drugs;
2) Brain or meningeal metastasis;
3) Previous allogeneic organ transplantation or allogeneic bone marrow transplantation;
4) The expected survival time is less than 12 weeks;
5) Chronic hepatitis B (HbsAg or HBcAb positive and HBV DNA ≥ 1000 IU/mL), hepatitis C (HCV antibody positive and HCV RNA quantitative detection is higher than the lower limit of the detection value of the research center), HIV antibody positive;
6) There is previous anti-tumor treatment toxicity > grade 1 (except for hair loss, pigmentation or other toxicity that is considered to have no safety risk to the subjects in the study);
7) Active bacterial infection, fungal infection or viral infection requiring systemic treatment within 1 week before the first administration;
8) Local or systemic anti-tumor therapy is received within 4 weeks before the first administration (2 weeks before administration for traditional Chinese medicine or Chinese patent medicine);
9) Other clinical trial drugs are received within 4 weeks before the first administration;
10) Serious thrombosis within the past 6 months, including pulmonary embolism, visceral embolism, deep vein thrombosis, etc.;
11) Other malignant active tumors in the past 3 years, except for locally curable cancers that have been cured, such as basal or squamous cell skin cancer, superficial bladder cancer or in situ prostate, cervical or breast cancer;
12) Abnormal cardiac function, including:
   · Long QTc syndrome or QTc interval >480ms;
   · Complete left bundle branch block, degree II or III atrioventricular block (except for treatment by pacemaker implantation);
   · Severe, uncontrolled cardiac arrhythmias requiring medical treatment;
   · History of chronic congestive heart failure, and NYHA ≥ grade 3;
   · Left ventricular ejection fraction less than 50% within the past 6 months;
   · Valvular heart disease with CTCAE ≥ grade 3;
   · Uncontrollable hypertension (defined as multiple measurements of systolic blood pressure ≥160 mmHg or diastolic blood pressure ≥100 mmHg under medication control);
   · History of myocardial infarction, unstable angina, severe pericardial disease, acute ischemia, or electrocardiographic evidence of severe active conduction system abnormalities within 6 months before screening.
13) Adriamycin or other anthracycline therapy has been received, and the cumulative dose of adriamycin exceeds 400 mg/m² (anthracycline equivalent dose calculation: 1 mg adriamycin = 2mg epirubicin = 2mg daunorubicin = 0.5 mg idarubicin = 0.45 mg mitoxantrone); the cumulative dose of adriamycin liposome exceeds 900 mg/m²;
14) Pregnant or lactating women;
15) Suffering from any serious and/or uncontrollable disease that, as determined by investigators, other diseases that may affect other diseases (including but not limited to, uncontrolled diabetes mellitus, kidney disease requiring dialysis, severe liver disease) diseases, life-threatening autoimmune and bleeding disorders, substance abuse, neurological disorders, etc.);
16) Other conditions unsuitable to participate judged by investigators.

### (3) Criteria for withdrawal from treatment and research

The Subject must stop receiving the investigational drug treatment if any of the following conditions occurs during the study:
1) The subject has intolerable toxicity, and the investigator evaluates that the risks of continuing to receive the treatment outweigh the benefits;
2) Concomitant diseases that occur during treatment do not allow subsequent continued administration;
3) Disease progression assessed by imaging;
4) There is clinically assessed disease progression or major protocol deviation, or poor compliance of the subject, and it is judged by the investigator that the continued treatment with the treatment will not benefit;
5) The subject is pregnant;
6) Death;
7) Meet any of the withdrawal criteria.

All subjects who withdraw from treatment are required to continue follow-up in accordance with the study plan, except for those who discontinue treatment due to death or who meet any of the following withdrawal criteria.

Subjects have the right to withdraw from the study at any time for any reason. Subjects will be withdrawn from the study process if any of the following occurs:
1) Lost to follow-up;
2) Subject or family members request to withdraw from the trial;
3) Termination of the study;
4) Other.

### 3. Research results

For subjects with no chance of radical surgery or metastatic bone and soft tissue sarcoma, after the first-line standard treatment, the second-line treatment drugs were not effective. Therefore, improving the treatment had always been a difficult clinical problem, and new treatments were urgently needed to be explored. In the dose escalation study (HE071-01), mitoxantrone liposome had shown certain curative effect in 12 mg/m² and 14 mg/m² groups of advanced soft tissue sarcoma subjects. The inventors of the present invention used mitoxantrone liposomes. After the drug entered the human body through intravenous infusion, it had the effects of sustained release, targeting, detoxification and synergism. Not only was the dosage higher than that of ordinary injections, but it is also a single drug treatment. It could not only improve the effectiveness, but also reduce the incidence of adverse reactions.

The study of the present invention showed that among the evaluable subjects, among 3 subjects with osteosarcoma (2 cases received 2 cycles of treatment, 1 case received 1 cycle of treatment), 1 subject was evaluated as SD (1 cycle of treatment was received), DCR was 33.3% (1/3).

In soft tissue sarcoma, among 12 subjects (1 case subjected to 6 cycles of treatment, 2 cases subjected to 5 cycles of treatment, 2 cases subjected to 4 cycles of treatment, 1 case subj ected to 3 cycles of treatment, and 6 cases subj ected to 2 cycles of treatment) were evaluated as SD (1 case subjected to 6 cycles of treatment, 2 cases subjected to 5 cycles of treatment, 2 cases subjected to 4 cycles of treatment, 1 case subjected to 3 cycles of treatment, and 2 cases subjected to 2 cycles of treatment; 3 subjects had tumor volume reduction), DCR was 66.7% (8/12).

Mitoxantrone liposome had good curative effect in patients with osteosarcoma and soft tissue sarcoma. 9 subjects who were evaluated as SD continued the treatment and obtained better curative effect.

The embodiments of the technical solutions of the present invention have been exemplarily described above. It should be understood that the protection scope of the present invention is not limited to the above-mentioned embodiments. Any modification, equivalent replacement, improvement, etc. made by those skilled in the art within the spirit and principle of the present invention shall be included within the protection scope of the claims of the present application.

## Claims

1. Mitoxantrone hydrochloride liposome for use in a method for treating urothelial carcinoma, the method is to administer a therapeutically effective amount of mitoxantrone hydrochloride liposome to a patient suffering from urothelial carcinoma; wherein the urothelial cancer is locally advanced or metastatic urothelial carcinoma; and
wherein the patient suffering therefrom is subjected to treatment-failure via platinum containing chemotherapy regimens and/or PD-1 inhibitors.

2. Mitoxantrone hydrochloride liposome as the only active ingredient for use in a method for treating urothelial carcinoma, the method is to administer a therapeutically effective amount of mitoxantrone hydrochloride liposome to a patient suffering from urothelial carcinoma; wherein the urothelial cancer is locally advanced or metastatic urothelial carcinoma; and
wherein the patient suffering therefrom is subjected to treatment-failure via platinum containing chemotherapy regimens and/or PD-1 inhibitors.

3. Mitoxantrone hydrochloride liposome for use in a method according to claim 1 or 2, **characterized in that** the mitoxantrone hydrochloride liposome is in the form of injection, including liquid injection, powder for injection, tablet for injection.

4. Mitoxantrone hydrochloride liposome for use in a method according to claim 1 or 2, **characterized in that** the mitoxantrone hydrochloride liposome is a liquid injection.

5. Mitoxantrone hydrochloride liposome for use in a method according to claim 1 or 2, **characterized in that** the mitoxantrone hydrochloride liposome contains 0.5-5 mg/ml, or 1-2 mg/ml, or 1 mg/ml of active ingredient, based on mitoxantrone.

6. Mitoxantrone hydrochloride liposome for use in a method according to claim 1 or 2, **characterized in that** the administration method is an intravenous administration.

7. Mitoxantrone hydrochloride liposome for use in a method according to claim 6, **characterized in that** for each intravenous administration, the infusion administration time of the liposome pharmaceutical preparation is 30 min-120 min, or 60 min-120 min, or 60±15 min.

8. Mitoxantrone hydrochloride liposome for use in a method according to claim 1 or 2, **characterized in that** the administration cycle is once every 4 weeks or once every 3 weeks.

9. Mitoxantrone hydrochloride liposome for use in a method according to claim 1 or 2, **characterized in that** the therapeutically effective amount is 8-30 mg/m² based on mitoxantrone.

10. Mitoxantrone hydrochloride liposome for use in a method according to claim 1 or 2, **characterized in that** the therapeutically effective amount is 12-20 mg/m², based on mitoxantrone, or the therapeutically effective amount is 20 mg/m², based on mitoxantrone.

11. Mitoxantrone hydrochloride liposome for use in a method according to claim 1 or 2, **characterized in that** the mitoxantrone hydrochloride liposome has one or more of the following properties:
(i) the mitoxantrone hydrochloride liposome has a particle size of 30-80 nm;
(ii) the mitoxantrone hydrochloride forms an insoluble precipitate with multivalent counter ions (e.g. sulfate, citrate or phosphate) in the liposome;
(iii) the phospholipid bilayer of the mitoxantrone hydrochloride liposome contains a phospholipid with a phase transition temperature (Tm) higher than body temperature, such that the phase transition temperature of the liposome is higher than body temperature, e.g., the phospholipid is selected from hydrogenated soy lecithin, phosphatidylcholine, hydrogenated ovolecithin, lecithin bis palmitate, lecithin bis stearate, or any combination thereof;
(iv) the phospholipid bilayer of the mitoxantrone hydrochloride liposome contains hydrogenated soy lecithin, cholesterol, and distearoylphosphatidylethanolamine modified with polyethylene glycol 2000 (DSPE-PEG2000);
(v) the phospholipid bilayer of the mitoxantrone hydrochloride liposome contains hydrogenated soy lecithin, cholesterol and distearoylphosphatidylethanolamine modified with polyethylene glycol 2000 in a mass ratio of 3:1:1, the mitoxantrone hydrochloride forms an insoluble precipitate with the multivalent acid anion in the liposome, and the particle size of the mitoxantrone hydrochloride liposome in the drug is 60 nm.

12. Mitoxantrone hydrochloride liposome for use in a method according to claim 11, **characterized in that** the mitoxantrone hydrochloride liposome contains: 1) the active ingredient mitoxantrone, which can form an insoluble precipitate with polyvalent counter ions in the liposome, 2) the phospholipid bilayer contains a phospholipid with a phase transition temperature (Tm) higher than body temperature; the phospholipid with the Tm higher than body temperature is phosphatidylcholine, hydrogenated soy lecithin, hydrogenated ovolecithin, lecithin bis palmitate or lecithin bis stearate, or any combination thereof.

13. Mitoxantrone hydrochloride liposome for use in a method according to claim 11, **characterized in that** the particle size of the mitoxantrone hydrochloride liposome is 35-75 nm, or the particle size of the mitoxantrone hydrochloride liposome is 40-70 nm, or the particle size of the mitoxantrone hydrochloride liposome is 40-60 nm, or the particle size of the mitoxantrone hydrochloride liposome is 60 nm.

14. Mitoxantrone hydrochloride liposome for use in a method according to claim 11, **characterized in that** the phospholipid bilayer contains hydrogenated soy lecithin, cholesterol and distearoylphosphatidylethanolamine modified with polyethylene glycol 2000 in a mass ratio of 3:1:1, the particle size is 60 nm, the counter ion is sulfate ion.

15. Mitoxantrone hydrochloride liposome for use in a method according to claim 11, **characterized in that** the phospholipid bilayer of the liposome contains hydrogenated soy lecithin, cholesterol and distearoylphosphatidylethanolamine modified with polyethylene glycol 2000 in a mass ratio of 3:1:1, and the particle size is 40-60 nm, the counter ion is sulfate ion, and the weight ratio of HSPC : Chol : DSPE-PEG2000 : mitoxantrone in the liposome is 9.58 : 3.19 : 3.19 : 1.

## Patentansprüche

1. Mitoxantron-Hydrochlorid-Liposom zur Verwendung in einem Verfahren zur Behandlung eines Urothel-Karzinoms, wobei das Verfahren darin besteht, eine therapeutisch wirksame Menge an Mitoxantron-Hydrochlorid-Liposom an einen Patienten zu verabreichen, der an einem Urothel-Karzinom leidet; wobei der Urothel-Krebs lokal ein lokal fortgeschrittenes oder metastatisches Urothel-Karzinom ist; und
wobei der daran leidende Patient gegenüber einer Behandlung mit platinhaltigen Chemotherapie-Schemata und/oder PD-1-Inhibitoren therapieresistent ist.

2. Mitoxantron-Hydrochlorid-Liposom als einziger aktiver Bestandteil zur Verwendung in einem Verfahren zur Behandlung eines Urothel-Karzinoms, wobei das Verfahren darin besteht, eine therapeutisch wirksame Menge an Mitoxantron-Hydrochlorid-Liposom an einen Patienten zu verabreichen, der an einem Urothel-Karzinom leidet; wobei der Urothel-Krebs lokal ein lokal fortgeschrittenes oder metastatisches Urothel-Karzinom ist; und
wobei der daran leidende Patient gegenüber einer Behandlung mit platinhaltigen Chemotherapie-Schemata und/oder PD-1-Inhibitoren therapieresistent ist.

3. Mitoxantron-Hydrochlorid-Liposom zur Verwendung in einem Verfahren nach Anspruch 1 oder 2, **dadurch gekennzeichnet, dass** das Mitoxantron-Hydrochlorid-Liposom in der Form einer Injektion vorliegt, was eine flüssige Injektion, ein Pulver zur Injektion, eine Tablette zur Injektion beinhaltet.

4. Mitoxantron-Hydrochlorid-Liposom zur Verwendung in einem Verfahren nach Anspruch 1 oder 2, **dadurch gekennzeichnet, dass** das Mitoxantron-Hydrochlorid-Liposom eine flüssige Injektion ist.

5. Mitoxantron-Hydrochlorid-Liposom zur Verwendung in einem Verfahren nach Anspruch 1 oder 2, **dadurch gekennzeichnet, dass** das Mitoxantron-Hydrochlorid-Liposom 0,5 - 5 mg/ml, oder 1 - 2 mg/ml, oder 1 mg/ml des aktiven Bestandteils bezogen auf Mitoxantron enthält.

6. Mitoxantron-Hydrochlorid-Liposom zur Verwendung in einem Verfahren nach Anspruch 1 oder 2, **dadurch gekennzeichnet, dass** das Verfahren zur Verabreichung eine intravenöse Verabreichung ist.

7. Mitoxantron-Hydrochlorid-Liposom zur Verwendung in einem Verfahren nach Anspruch 6, **dadurch gekennzeichnet, dass** für jede intravenöse Verabreichung die Zeit für die Infusionsverabreichung der pharmazeutischen Liposom-Zubereitung 30 min - 120 min, oder 60 min - 120 min, oder 60 ± 15 min beträgt.

8. Mitoxantron-Hydrochlorid-Liposom zur Verwendung in einem Verfahren nach Anspruch 1 oder 2, **dadurch gekennzeichnet, dass** der Verabreichungszyklus einmal alle 4 Wochen oder einmal alle 3 Wochen ist.

9. Mitoxantron-Hydrochlorid-Liposom zur Verwendung in einem Verfahren nach Anspruch 1 oder 2, **dadurch gekennzeichnet, dass** die therapeutisch wirksame Menge 8 - 30 mg/m² bezogen auf Mitoxantron beträgt.

10. Mitoxantron-Hydrochlorid-Liposom zur Verwendung in einem Verfahren nach Anspruch 1 oder 2, **dadurch gekennzeichnet, dass** die therapeutisch wirksame Menge 12 - 20 mg/m² bezogen auf Mitoxantron beträgt, oder die therapeutisch wirksame Menge 20 mg/m² bezogen auf Mitoxantron beträgt.

11. Mitoxantron-Hydrochlorid-Liposom zur Verwendung in einem Verfahren nach Anspruch 1 oder 2, **dadurch gekennzeichnet, dass** das Mitoxantron-Hydrochlorid-Liposom eine oder mehrere der folgenden Eigenschaften aufweist:
(i) das Mitoxantron-Hydrochlorid-Liposom weist eine Partikelgröße von 30 - 80 nm auf;
(ii) das Mitoxantron-Hydrochlorid bildet mit mehrwertigen Gegenionen (e.g. Sulfat, Citrat oder Phosphat) in dem Liposom ein unlösliches Präzipitat;
(iii) die Phospholipid-Doppelschicht des Mitoxantron-Hydrochlorid-Liposoms enthält ein Phospholipid mit einer Phasenübergangstemperatur (Tm), die höher ist als die Körpertemperatur, so dass die Phasenübergangstemperatur des Liposoms höher ist als die Körpertemperatur, z. B. ist das Phospholipid ausgewählt aus hydriertem Sojalecithin, Phosphatidylcholin, hydriertem Ovolecithin, Lecithin-Bis-Palmitat, Lecithin-Bis-Stearat oder einer beliebigen Kombination davon;
(iv) die Phospholipid-Doppelschicht des Mitoxantron-Hydrochlorid-Liposoms enthält hydriertes Sojalecithin, Cholesterin und mit Polyethylenglykol 2000 (DSPE-PEG2000) modifiziertes Distearoylphosphatidylethanolamin;
(v) die Phospholipid-Doppelschicht des Mitoxantron-Hydrochlorid-Liposoms enthält hydriertes Sojalecithin, Cholesterin und mit Polyethylenglykol 2000 modifiziertes Distearoylphosphatidylethanolamin in einem Massenverhältnis von 3 : 1 : 1, das Mitoxantron-Hydrochlorid bildet mit dem mehrwertigen Säure-Ion in dem Liposom ein unlösliches Präzipitat, und die Partikelgröße des Mitoxantron-Hydrochlorid-Liposoms in dem Arzneistoff beträgt 60 nm.

12. Mitoxantron-Hydrochlorid-Liposom zur Verwendung in einem Verfahren nach Anspruch 11, **dadurch gekennzeichnet, dass** das Mitoxantron-Hydrochlorid-Liposom enthält: 1) den aktiven Bestandteil Mitoxantron, der mit mehrwertigen Gegenionen in dem Liposom ein unlösliches Präzipitat bilden kann, 2) die Phospholipid-Doppelschicht enthält ein Phospholipid mit einer Phasenübergangstemperatur (Tm), die höher ist als die Körpertemperatur; das Phospholipid mit der Tm, die höher ist als die Körpertemperatur ist Phosphatidylcholin, hydriertes Sojalecithin, hydriertes Ovolecithin, Lecithin-Bis-Palmitat oder Lecithin-Bis-Stearat, oder eine beliebige Kombination davon.

13. Mitoxantron-Hydrochlorid-Liposom zur Verwendung in einem Verfahren nach Anspruch 11, **dadurch gekennzeichnet, dass** die Partikelgröße des Mitoxantron-Hydrochlorid-Liposoms 35 - 75 nm beträgt, oder die Partikelgröße des Mitoxantron-Hydrochlorid-Liposoms 40 - 70 nm beträgt, oder die Partikelgröße des Mitoxantron-Hydrochlorid-Liposoms 40 - 60 nm beträgt, oder die Partikelgröße des Mitoxantron-Hydrochlorid-Liposoms 60 nm beträgt.

14. Mitoxantron-Hydrochlorid-Liposom zur Verwendung in einem Verfahren nach Anspruch 11, **dadurch gekennzeichnet, dass** die Phospholipid-Doppelschicht hydriertes Sojalecithin, Cholesterin und mit Polyethylenglykol 2000 modifiziertes Distearoylphosphatidylethanolamin in einem Massenverhältnis von 3 : 1 : 1 enthält, die Partikelgröße 60 nm beträgt und das Gegenion ein Sulfat-Ion ist.

15. Mitoxantron-Hydrochlorid-Liposom zur Verwendung in einem Verfahren nach Anspruch 11, **dadurch gekennzeichnet, dass** die Phospholipid-Doppelschicht hydriertes Sojalecithin, Cholesterin und mit Polyethylenglykol 2000 modifiziertes Distearoylphosphatidylethanolamin in einem Massenverhältnis von 3 : 1 : 1 enthält, und die Partikelgröße 40 - 60 nm beträgt, das Gegenion ein Sulfat-Ion ist und das Gewichtsverhältnis von HSPC : Chol : DSPE-PEG2000 : Mitoxantron in dem Liposom 9,58 : 3,19 : 3,19 : 1 beträgt.

## Revendications

1. Liposome de chlorhydrate de mitoxantrone destiné à être utilisé dans une méthode de traitement d'un carcinome urothélial, la méthode consistant à administrer une quantité thérapeutiquement efficace de liposome de chlorhydrate de mitoxantrone à un patient souffrant d'un carcinome urothélial ; dans lequel le cancer urothélial est un carcinome urothélial localement avancé ou métastasique ; et
dans lequel le patient souffrant de celui-ci est sujet à un échec de traitement par l'intermédiaire de régimes de chimiothérapie contenant du platine et/ou d'inhibiteurs de PD-1.

2. Liposome de chlorhydrate de mitoxantrone en tant qu'unique ingrédient actif destiné à être utilisé dans une méthode de traitement d'un carcinome urothélial, la méthode consistant à administrer une quantité thérapeutiquement efficace de liposome de chlorhydrate de mitoxantrone à un patient souffrant d'un carcinome urothélial ; dans lequel le cancer urothélial est un carcinome urothélial localement avancé ou métastasique ; et
dans lequel le patient souffrant de celui-ci est sujet à un échec de traitement par l'intermédiaire de régimes de chimiothérapie contenant du platine et/ou d'inhibiteurs de PD-1.

3. Liposome de chlorhydrate de mitoxantrone destiné à être utilisé dans une méthode selon la revendication 1 ou 2, **caractérisé en ce que** le liposome de chlorhydrate de mitoxantrone se présente sous la forme d'une injection, comprenant une injection liquide, une poudre pour injection, un comprimé pour injection.

4. Liposome de chlorhydrate de mitoxantrone destiné à être utilisé dans une méthode selon la revendication 1 ou 2, **caractérisé en ce que** le liposome de chlorhydrate de mitoxantrone est une injection liquide.

5. Liposome de chlorhydrate de mitoxantrone destiné à être utilisé dans une méthode selon la revendication 1 ou 2, **caractérisé en ce que** le liposome de chlorhydrate de mitoxantrone contient 0,5 à 5 mg/ml, ou 1 à 2 mg/ml, ou 1 mg/ml d'ingrédient active, par rapport à la mitoxantrone.

6. Liposome de chlorhydrate de mitoxantrone destiné à être utilisé dans une méthode selon la revendication 1 ou 2, **caractérisé en ce que** la méthode d'administration est une administration intraveineuse.

7. Liposome de chlorhydrate de mitoxantrone destiné à être utilisé dans une méthode selon la revendication 6, **caractérisé en ce que** pour chaque administration intraveineuse le temps d'administration par perfusion de la préparation pharmaceutique de liposome est de 30 min à 120 min, ou de 60 min à 120 min ou de 60 ± 15 min.

8. Liposome de chlorhydrate de mitoxantrone destiné à être utilisé dans une méthode selon la revendication 1 ou 2, **caractérisé en ce que** le cycle d'administration est une fois toutes les 4 semaines ou une fois toutes les 3 semaines.

9. Liposome de chlorhydrate de mitoxantrone destiné à être utilisé dans une méthode selon la revendication 1 ou 2, **caractérisé en ce que** la quantité thérapeutiquement efficace est de 8 à 30 mg/m² par rapport à la mitoxantrone.

10. Liposome de chlorhydrate de mitoxantrone destiné à être utilisé dans une méthode selon la revendication 1 ou 2, **caractérisé en ce que** la quantité thérapeutiquement efficace est de 12 à 20 mg/m², par rapport à la mitoxantrone, ou la quantité thérapeutiquement efficace est de 20 mg/m², par rapport à la mitoxantrone.

11. Liposome de chlorhydrate de mitoxantrone destiné à être utilisé dans une méthode selon la revendication 1 ou 2, **caractérisé en ce que** le liposome de chlorhydrate de mitoxantrone présente une ou plusieurs des propriétés suivantes :
(i) le liposome de chlorhydrate de mitoxantrone présente une taille de particule de 30 à 80 nm ;
(ii) le chlorhydrate de mitoxantrone forme un précipité soluble avec des contre-ions multivalents (par ex. sulfate, citrate ou phosphate) dans le liposome ;
(iii) la bicouche phospholipidique du liposome de chlorhydrate de mitoxantrone contient un phospholipide avec une température de transition de phase (Tm) supérieure à la température corporelle, de sorte que la température de transition de phase du liposome soit supérieure à la température corporelle, par ex., le phospholipide est choisi parmi la lécithine de soja hydrogénée, la phosphatidylcholine, l'ovolécithine hydrogénée, le bis palmitate de lécithine, le bis stéarate de lécithine, ou toute combinaison de ceux-ci ;
(iv) la bicouche phospholipidique du liposome de chlorhydrate de mitoxantrone contient de la lécithine de soja hydrogénée, du cholestérol et de la distéaroylphosphatidyléthanolamine modifiée avec du polyéthylène glycol 2000 (DSPE-PEG2000) ;
(v) la bicouche phospholipidique du liposome de chlorhydrate de mitoxantrone contient de la lécithine de soja hydrogénée, du cholestérol et de la distéaroylphosphatidyléthanolamine modifiée avec du polyéthylène glycol 2000 en un rapport massique de 3:1:1, le chlorhydrate de mitoxantrone forme un précipité insoluble avec l'anion d'acide multivalent dans le liposome, et la taille de particule du liposome de chlorhydrate de mitoxantrone dans le médicament est de 60 nm.

12. Liposome de chlorhydrate de mitoxantrone destiné à être utilisé dans une méthode selon la revendication 11, **caractérisé en ce que** le liposome de chlorhydrate de mitoxantrone contient : 1) l'ingrédient actif mitoxantrone, qui peut former un précipité insoluble avec les contre-ions polyvalents dans le liposome, 2) la bicouche phospholipidique contient un phospholipide avec une température de transition de phase (Tm) supérieure à la température corporelle, le phospholipide avec la Tm supérieure à la température corporelle est la phosphatidylcholine, la lécithine de soja hydrogénée, l'ovolécithine hydrogénée, le bis palmitate de lécithine ou le bis stéarate de lécithine, ou toute combinaison de ceux-ci.

13. Liposome de chlorhydrate de mitoxantrone destiné à être utilisé dans une méthode selon la revendication 11, **caractérisé en ce que** la taille de particule du liposome de chlorhydrate de mitoxantrone est de 35 à 75 nm, ou la taille de particule du liposome de chlorhydrate de mitoxantrone est de 40 à 60 nm, ou la taille de particule du liposome de chlorhydrate de mitoxantrone est de 60 nm.

14. Liposome de chlorhydrate de mitoxantrone destiné à être utilisé dans une méthode selon la revendication 11, **caractérisé en ce que** la bicouche phospholipidique contient de la lécithine de soja hydrogénée, du cholestérol et de la distéaroylphosphatidyléthanolamine modifiée avec du polyéthylène glycol 2000 en un rapport massique de 3:1:1, la taille de particule est de 60 nm, le contre-ion est l'ion sulfate.

15. Liposome de chlorhydrate de mitoxantrone destiné à être utilisé dans une méthode selon la revendication 11, **caractérisé en ce que** la bicouche phospholipidique du liposome contient de la lécithine de soja hydrogénée, du cholestérol et de la distéaroylphosphatidyléthanolamine modifiée avec du polyéthylène glycol 2000 en un rapport massique de 3:1:1, et la taille de particule est de 40 à 60 nm, le contre-ion est l'ion sulfate et le rapport massique de HSPC : Chol : DSPE-PEG2000 : mitoxantrone dans le liposome est de 9,58 : 3,19 : 3,19 : 1.
